# EUROPEAN PATENT SPECIFICATION

(11) **EP 3 948 352 B1**
(45) Date of publication and mention of the grant of the patent: **10.12.2025**
(21) Application number: 20713507.0
(22) Date of filing: 02.04.2020
(51) Int. Cl.: G01T 1/02

(54) **SYSTEM AND METHOD FOR MONITORING RADIATION EXPOSURE IN A HOSPITAL ENVIRONMENT FOR AN OPERATOR**
SYSTEM UND VERFAHREN ZUR ÜBERWACHUNG DER STRAHLENBELASTUNG IN EINER KRANKENHAUSUMGEBUNG FÜR EINE BEDIENPERSON
SYSTÈME ET PROCÉDÉ DE SURVEILLANCE DE L'EXPOSITION AU RAYONNEMENT DANS UN ENVIRONNEMENT HOSPITALIER POUR UN OPÉRATEUR

(30) Priority: 05.04.2019 IT 201900005170
(43) Date of publication of application: 09.02.2022
(73) Proprietor: Ubiquicom S.r.l., 20141 Milano (IT); Fismeco S.r.l., 00159 Roma (IT)
(72) Inventor: INDOVINA, Luca, 00196 Roma (IT); SARASSO, Stefano, 20133 Milano (IT)
(74) Representative: Tansini, Elio Fabrizio
(86) International application number: PCT/IB2020/053144
(87) International publication number: WO 2020/202061

(56) References cited:
- EP-A1- 3 451 277
- US-A1- 2012 235 064
- US-A1- 2014 312 242

## Description

### Technical Field

The present invention relates to a system and method for monitoring radiation exposure in a hospital environment for an operator.

In particular, the present invention relates to a method and system for monitoring the radiation exposure of an operator wearing a dosimeter and operating within a hospital area where ionizing radiation exposure is present.

### Prior Art

As is well known, the traditional dosimeters (both passive and active) currently used in hospitals and in particular in operating theatres (for example in Cardiology, Endoscopy, Therapy, General Surgery, Orthopaedics) present numerous problems that have not yet been solved, as they only allow measuring the ionizing radiation to which they are exposed.

Ionizing radiation comprises high energy electromagnetic waves (X-rays, gamma rays) and particles (α particles, β particles, neutrons). Ionizing radiation is emitted by radioactive elements and instruments such as X-ray apparatuses and radiation therapy equipment.

Most imaging methods using ionizing radiation (e.g., x-rays, CT, scintigraphy) expose patients to relatively low doses of radiation, generally considered harmless. However, any dose of ionizing radiation is potentially harmful, and there is no threshold below which no harmful effects occur, so every effort is made to minimize exposure to ionizing radiation.

To date, the radiation detection devices worn by an operator do not allow:
- ensuring that they are actually worn by a specific operator (e.g. the personal dosimeter could simply be "placed" near the radiation source but not worn), and that, if worn, they are actually worn by the specific operator to which they are assigned, and positioned correctly (e.g. at chest height);
- verifying that the prescribed Personal Protective Equipment (PPE) is also worn at the same time;
- knowing and monitoring the operator's actual distance from the radiation source, and therefore compliance with the prescribed minimum distances;
- detecting and intervening in real time in the event of abnormal situations;
- measuring how long the apparatuses are actually used;
- measuring how much radiation is emitted during a specific procedure;
- collecting detailed data on time periods that can be parametrized (Big Data) and related to all personnel involved, to allow Management to carry out precise and in-depth analyses and identify critical issues and related opportunities for improvement as well as for safety during exposure, including, for example, checks on the number of operators actually present during the service in the operating theatre, how long an average service lasts, how much an apparatus is actually used;

The known passive dosimeters allow data to be stored in the dosimeter itself (which is not connected) and communicated centrally only on a periodic basis (typically once a month), often manually, with the consequent possibility of making errors or omissions (e.g. to improperly benefit from the radiological risk allowances that in Italy legally envisage 15 days of holiday/year and a remuneration of 100 euros/month in relation to the activities carried out);
The known type of electronic dosimeters allow the operator to obtain an immediate reading of the radiation exposure, but the same is in no way centrally stored and its reading can however distract the operator during normal activity within the hospital area.

Only traditional dosimeters are used in Italy and abroad, which are not able to solve even one of the problems mentioned above.

One type of known dosimeters envisages electronic dosimeters connected to a traffic light in the operating theatre that indicates to the operator, during performance, the possible exceeding of a reference exposure level (red traffic light).

However, this solution does not allow constantly monitoring the operator's position, working conditions, exposure time or whether Personal Protective Equipment (PPE) is worn correctly.

One drawback of the passive dosimeters and direct-read dosimeters is that they provide a simple exposure measurement that is not centrally stored and lacks any information on the place and mode of work in which the operator has been exposed to ionizing radiation (location, position with respect to the X-ray source, exposure time, PPE used).

EP 3 451 277 A1 and US 2012/235064 A1 disclose prior art for the claimed invention.

### Object of the invention

An object of the present invention is to detect and monitor in real time the exposure to ionizing radiation (i.e. effective and/or equivalent dose) received by the operator during ionizing radiation activities (in particular in operating rooms to ensure verification of the dose received during the activity) and the consequent failure to exceed the dose limits.

Another object of the present invention is to detect and monitor in real time the correct position (distance) of the operator with respect to the radiation source during performance, as optimizing the operator/source distance allows minimizing the doses for the operator himself as well as the exposure time in the individual activity.

A further object of the present invention is to detect and monitor in real time the presence of Personal Protective Equipment (PPE) worn by the operator to ensure the correct execution of risk containment procedures.

Another object of the present invention is to detect and monitor in real time the actual use of the dosimeter worn by the operator, also to prevent fraudulent behaviour as much as possible.

A further object of the present invention is to detect and monitor in real time the verification of the operators actually present during operating theatre services and the amount of time in which the apparatuses are actually used.

Another object of the present invention is that of providing a method and system for monitoring exposure to radiation in a hospital environment that can guarantee a high level of safety.

A further object of the present invention is that of providing a method and system for monitoring exposure to radiation in a hospital environment that is in real time and efficient.

A further and not least object of the present invention is that of providing a method and system for monitoring exposure to radiation in a hospital environment that is highly reliable, easy to realize and simple to use.

In a first aspect of the invention, the above-mentioned objects are achieved by a system for monitoring exposure to radiation in a hospital environment according to that which is disclosed in claim 1.

Advantageous aspects are disclosed in dependent claims 2 to 12.

In a second aspect of the invention, the above-mentioned objects are achieved by an accommodation facility access method according to that which is disclosed in claim 13.

In general, the invention offers the following technical effects:
- allows an increase in the safety of operators within hospital areas subject to ionizing radiation;
- allows a reduction of the doses absorbed by the operators present within an area A;
- allows the personnel responsible for operator safety to implement procedures within the operating theatres for the various subjects/operators which optimise their interventions;
- allows real-time monitoring of the risk level of each operator present in the operating theatre;
- improves productivity and efficiency;
- allows reducing the possibility of mistakes or fraud;
- allows the real-time monitoring of the radiation emissions from medical apparatuses.

The technical effects/advantages mentioned, and other technical effects/advantages of the invention, will emerge in further detail from the description provided herein below of an example of embodiment provided by way of approximate and non-limiting example with reference to the appended drawings.

### Brief description of the drawings

For a better understanding of the invention and to appreciate the advantages thereof, several non-limiting example embodiments are described herein below, referring to the attached figures, in which:
- figure 1 is an overall view of the system of the invention.
- figure 2 is a block diagram of the system of figure 1.

### Detailed description of preferred embodiments of the invention

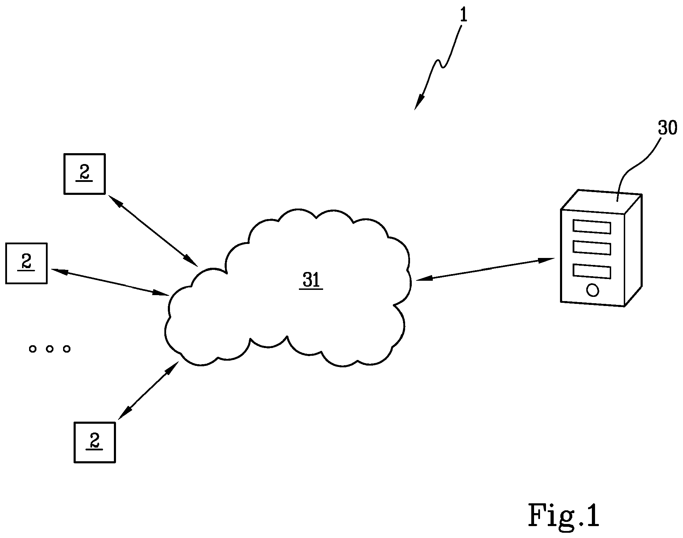

With reference to the cited figures, the system for monitoring exposure to radiation in a hospital environment according to the invention is indicated overall with the reference number 1 in the block diagram of figure 1.

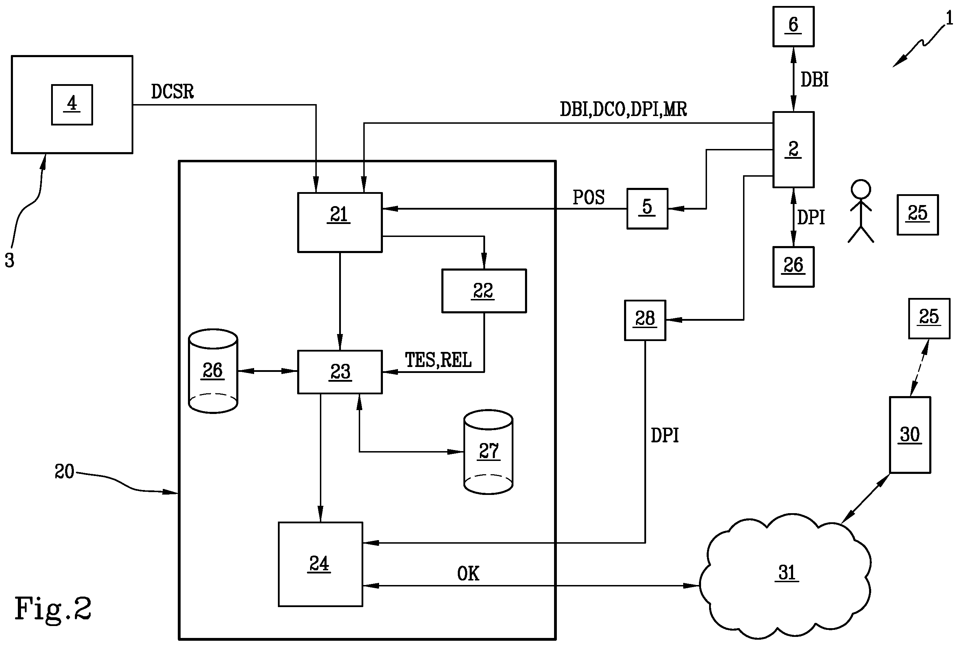

The system 1, shown in figures 1 and 2, comprises a dosimeter 2 wearable by a health care worker O, configured to measure the radiation MR to which the worker O is exposed in the hospital environment, a radiation source identification means 4 for identifying a source of radiation emitted by a medical or X-ray apparatus 3, comprising emitted radiation source identification data DCSR, the procedure (PRC) in which the radiation is used and the position of the radiation source (POA) within an area A of the hospital environment, a position acquisition means 5 for acquiring the position of the dosimeter 2, configured to communicate with said dosimeter 2 and to acquire the position in which it is located POS, a processing unit 20 configured to process data relating to the safety of each health care worker O and means for transmitting information and/or the conformity signal to a remote server 30.

The present invention allows the medical apparatuses 3, radiation sources, and workers present in a hospital area A to be managed safely and efficiently.

The invention further allows some hospital health safety procedures to be made more effective and simple and assistance to be provided with the performance of the related procedures.

The dosimeter 2 is configured to measure the dose in terms of personal dose equivalent Hp(10) (radiation protection magnitude related to the overall health risk to the worker due to exposure to ionizing radiation). The effective dose is expressed in Sievert units (Sv) and submultiples thereof such as millisv (mSv). The dosimeter 2 could be configured to measure the dose at the ends, such as hands or crystalline, in terms of personal dose equivalent Hp(0.07), always in units of mSv. The magnitudes Hp(10) and Hp(0.07) allow an easy evaluation of the equivalent dose and effective dose radiation protection magnitudes with which they correlate. The equivalent dose is the absorbed dose, defined as the energy released by the radiation per mass unit, multiplied by a specific weighting factor for the type of radiation, which takes into account the effects on tissues based on the type of radiation delivered (e.g. X-rays, gamma rays, electrons). It is also expressed in sievert (Sv) or its submultiples such as millisievert (mSv). The effective dose is instead an estimate of the probabilistic risk of the worker sustaining damage over his lifetime in relation to the effective dose value attributed thereto; it takes into account the equivalent dose based on the sensitivity of the tissue exposed to radiation (e.g. the gonads are more sensitive). The effective dose is also expressed in Sv or its submultiples (mSv).

The dosimeter 2 contains therein a unique identification number (ID), stored in a read-only internal memory, the circuitry for the measurement of ionizing radiation and a means for communicating the measured radiation MR in short and medium range and the unique identification number ID.

Preferably, each dosimeter 2 is personal and is assigned to a specific health care worker O. When the dosimeter 2 is assigned to a health care worker, a pairing operation is performed, and the relative record is stored in a first memory unit 26 of the system. In this way, each record of the memory unit 26 will contain in a first field the unique identifier ID of the dosimeter, and in a second field the characteristic data of the health care worker DCO. In this way a logical association is made between personal dosimeter 2 and worker.

The processing unit 20 and the remote server 30 are provided with interfaces that allow them to communicate with each other through an electronic communication network 31.

Figure 2 illustrates a non-limiting example of the invention, in which the processing unit 20 is arranged locally and the server 30 remotely. However, other non-limiting examples of the present invention contemplate the processing unit 20 and the server 30 arranged both locally or both remotely. Furthermore, in a further non-limiting embodiment of the present invention, the processing unit 20 and the server 30 coincide in a single device arranged locally or remotely.

The electronic network 31 is preferably the internet, but could also be an intranet network or any private network adapted to implement a client-server type communication protocol. The electronic network 31 is connected, where necessary, to mobile networks for communication between the remote server 30 and the processing unit 20.

According to the invention, the system 1 comprises a processing unit 20.

The processing unit 20 comprises at least one input module 21 configured to receive from the dosimeter 2 the unique identification number (ID) and the measurement of ionizing radiation MR to which it is exposed while operating within an environment A in which a radiation source 3 is present. The input module 21 receives from the radiation source identification means 4 characteristic data of the source of radiation emitted DCSR, data of the medical procedure delivered PRC in which it is used and the position POA of the medical or X-ray apparatus 3 (capable of emitting radiation) and receives from the position acquisition means 5, the position POS where the personal dosimeter 2 worn by the worker O is located.

A non-limiting example of a medical procedure delivered is coronary angioplasty, a radio-guided neurosurgery procedure to arrange a decomposed fracture of a limb.

The processing unit 20 further comprises a comparison module 23 configured to compare the data received by the input module 21 with corresponding predefined values stored in a second memory unit 27, such as for example average exposure time values for standard procedure, as well as average distances of a worker in said procedure or average effective dose and equivalent dose values in the procedures themselves.

The processing unit 20 comprises a conformity module 24 configured to generate a conformity signal as a function of a correspondence OK that has occurred in the comparison performed by the comparison module 23.

In general, it should be noted that in the present context and in the subsequent claims, the processing unit 20 is considered to be split into distinct functional modules (storage modules or operating modules) for the sole purpose of describing its functionalities clearly and completely.

Such processing unit can comprise a single electronic device, appropriately programmed to perform the functionalities described, and the different modules can correspond to hardware entities and/or routine software that are part of the programmed device.

Alternatively or additionally, these functionalities can be performed by a plurality of electronic devices on which the aforesaid functional modules can be distributed.

The processing unit 20 can also make use of one or more processors for executing the instructions contained in the memory modules.

The system 1 according to the invention advantageously comprises a display module 25 configured to generate a graphical representation or map of the radiation doses in the various positions within the hospital area A by the operator O during an intervention or session in the presence of radiation sources.

The map can be made available and displayed to the worker O, for example on a personal electronic device (such as a tablet or smartphone) or on a screen in the area A, or by another person located outside the area A, for example on the remote server 30 or other remote personal device.

The dosimeter 2, the radiation source identification means 4 for identifying a source of radiation emitted by the medical or X-ray apparatus 3, the position acquisition means 5 of the dosimeter 2 are able to wirelessly communicate with the processing unit 20 through a plurality of wireless access points that are in electronic communication with the processing unit 20.

The plurality of wireless access points will be located within the area A and will be configured not only to receive the wireless signals that are transmitted by the dosimeter 2, by the radiation source identification means 4, and by the position acquisition means 5, but also to communicate wireless signals to such devices.

In addition, the number of wireless access points is able to detect the specific location within area A, for example, of the dosimeter 2 and/or medical or X-ray apparatus 3 at any time.

For example, the dosimeter 2 periodically measures, at predetermined time intervals, the dose of ionizing radiation per time unit within the area A and communicates this accrual information to the processing unit 20, for example, communicates such accrual data to the wireless access points for communication to the processing unit 20.

At the same time, the wireless access points detect the position of the worker O (given by the position of the dosimeter 2 donned) along an x-axis, and a y-axis, and potentially along a z-axis, within the area A, detecting the position of the dosimeter 2 and/or the medical or X-ray apparatus 3.

Advantageously, the position acquisition means 5 for acquiring the position of the dosimeter 2 comprises one or more between, at least, time difference of arrival and/or two way ranging and/or angle of arrival and/or distance measurement and/or RSSI measurement (Received Signal Strength Indicator), on technologies such as optical or acoustic (e.g. ultrasound) radio frequency.

Advantageously, the position of the worker O is given by a location tag arranged on the dosimeter 2 or on the personal identification badge of the worker O.

The information which can be stored (at regular or selected time intervals) in a memory unit comprises, for example, the amount of radiation dose absorbed, detected by the dosimeter 2, the position in which the dosimeter is located is at the time of measurement, the position of the radiation source 4, optionally, the radiation emitted by the medical or X-ray apparatus 3 at the same time, and further includes a time stamp reflecting the time when such measurement was made.

The positions occupied by a particular worker O within a certain area A will be the points within the area A where the various radiation dosage measurements were recorded.

Alternatively, the system records and displays on the map the relative distances between a given worker O and the radiation source 4 emitted.

The measured doses can be displayed in numerical form on the map or by means of graphical representations such as different colours for the different intensities of absorbed radiation.

Dose intensity data can be taken directly from the measured dose intensity data set, or they can be normalised or otherwise processed.

The graphic representation thus obtained could be displayed to the operator himself, for his personal training, information and feedback, or it could be displayed or made available to a supervisor or technician or other individual interested in employee safety in such a way as to trace an exit and/or entry path from an area A for the worker O along an optimized path to minimize radiation exposure.

In this way, the invention allows minimizing the dose absorbed by each operator present in the hospital area A during a given treatment or operation.

The processing unit 20 comprises a calculation module 22 configured to calculate a radiation exposure time TES of the worker O and/or the relative distance REL between the position POS of the dosimeter 2 and the medical or X-ray apparatus 3, using the position data arriving at the position acquisition means 5.

Advantageously, the comparison module 23 is configured to compare the exposure time TES and/or said relative distance REL with predefined values stored in a second memory unit 27.

The dosimeter 2 wearable by the operator O according to the invention can advantageously comprise a biometric data DBI detector device 6 (e.g. fingerprints, colour and size of the iris, the retina, the shape of the hand, the palm of the hand, the shape of the ear, the physiognomy of the face, the tone of voice, etc.). In this case, the processing unit 20 is configured to make a comparison between at least one biometric data DBI of the health care worker O and the characteristic data of the worker DCO at the time the dosimeter 2 is donned. This coupling, biometric data detected DBI of a worker O and personal dosimeter 2, can be stored in a memory unit.

The biometric data detected DBI is advantageously sent to the input module 21 and the comparison module 24 will verify that the worker O is actually authorized to access the area A during a given procedure PRC.

In the invention, a module 28 is present for detecting the personal protective equipment worn by the worker O. This detection module 28 can be provided directly within the dosimeter 2 or outside it. An example of a detection technique is the ability of the detection module to read passive tags (e.g. RFID) applied to said PPE.

Once whether or not the operator is wearing the assigned personal protection equipment PPE and/or the type of personal protection equipment PPE worn by the worker O is detected, this data is advantageously sent to the input module 21 and, subsequently, the comparison module 23 will verify that the operator O is wearing the personal protection equipment PPE assigned and necessary to carry out a certain procedure PRC within the area A.

The characteristic data of the worker DCO can comprise one or more of, at least, personal data; identification data; biometric data; and/or health data. Preferably, the characteristic data of the emitted radiation source DCSR can comprise one or more between at least identification data; and/or ionizing radiation emission data.

The area A of the hospital environment is a radiologically controlled area located within the health care environment.

Advantageously, the position acquisition means 5 is arranged to detect the position POS of at least one between the dosimeter 2, the badge of the worker O and/or the medical or X-ray apparatus 3 located within the area A of the hospital environment.

Optionally, the health care worker's badge can contain a local electronic device containing its own characteristic data (DCO) and is capable of dialoguing with at least one between the dosimeter 2 and/or the position acquisition means 5 and/or the radiation exposure monitoring unit 19.

Preferably, the position acquisition means 5 is configured to store the position POS in association with the corresponding measured dose in a memory unit.

Preferably, placed in proximity to the zone of access to the area A there is an identification device 28 configured to identify the dosimeter 2 or the worker O entering or exiting the hospital area A or the personal identification badge.

Advantageously, the identification device 28 is in data connection with the processing unit 20, in particular, with the conformity module 24, and is able to send a significant signal PPE of whether or not the operator is wearing the required PPE.

If the worker O is not wearing the required personal protection equipment PPE, the conformity module 24 will generate a non-conformity signal KO, regardless of the outcome of the comparison carried out by the comparison module 23.

The identification means 4 of the medical or X-ray apparatus 3 consists of an RFID radio-frequency tag and/or an NFC tag and/or a barcode on various dimensions containing the characteristic data of the source of radiation emitted DCSR (for example, brand and model of the apparatus, date of the last revision, characteristic data of radiation emissions, etc.).

Optionally, an alarm module can be provided configured to send an alarm signal to the dosimeter 2 or to the electronic device given to the health care worker O as a function of a correspondence OK of the comparison made by said comparison module 23.

In a second aspect of the invention, a method for monitoring exposure to radiation in a hospital environment is provided comprising the steps of:
- assigning a wearable dosimeter 2 to a health care worker O, the dosimeter 2 being configured to measure the radiation MR to which the worker (O) is exposed and comprising a unique identification number ID;
- preparing on a medical or X-ray apparatus 3 a radiation source identification means 4 for identifying a source of radiation emitted by the apparatus 3 comprising data identifying the source of radiation emitted DCSR and the position of the apparatus 3 within an area A of the hospital environment;
- preparing a position acquisition means 5 for acquiring the position of the dosimeter 2, configured to communicate with said dosimeter 2 and to acquire the position in which it is located POS;
   - receiving the unique identification number ID and the measurement by said dosimeter 2 of the ionizing radiation MR to which he is exposed;
   - extracting from a first memory unit 26 characteristic data of the health care worker, using the unique identification number ID received from the dosimeter;
   - receiving said characteristic data of the source of radiation emitted DCSR by the medical or X-ray apparatus 3 from said means for identifying a source of radiation 4;
   - receiving the position POS in which the dosimeter 2 is located from said position acquisition means 5;
   - comparing the data received by the input module 21 with predefined values;
   - generating a conformity signal as a function of a correspondence OK that has occurred in the comparison performed;
   - transmitting the information and/or conformity signal to a remote server 30 via an information transmission means.
- providing a detection module (28) for detecting the personal protective equipment worn by the worker (O), configured to detect whether the worker (O) is wearing one or more items of personal protective equipment.

A third aspect of the present disclosure, not according to the wording of the claims, relates to a computer program that, when running on a computer, implements at least one or more steps of the method according to the first aspect of the invention.

The method according to the invention further comprises the functional characteristics of the operating modules described in the system 1.

The above allows:
▪ better safeguarding the safety of personnel through constant monitoring of radiation exposure and better scheduling of shifts, thus ensuring compliance with current legislation in terms of justification, optimisation and limitation of workers' exposure;
▪ avoiding criminal sanctions, even up to the suspension or revocation of authorisations for the exercise of activities with ionizing radiation, imposed by the control bodies (in Italy ASL (Local Health Authority), Ministry, Provincial Labour Directorate) in the presence of the incorrect management of radiation protection safety for workers, especially in operating theatres, where the risk of exposure is considered very high;
▪ avoiding those additional penalties typically paid when appropriate procedures involving the correct use of PPE are not documented.

As a person skilled in the art can easily understand, the invention allows overcoming the drawbacks highlighted above with reference to the prior art.

In particular, the present invention allows improving the safety of the workers and means operating in the zone with exposure to ionizing radiation. It also enables better safety management by the supervisor.

It is clear that the specific characteristics are described in relation to different embodiments of the invention with an exemplary and non-limiting intent. Obviously a person skilled in the art can make further modifications and variants to the present invention, in order to satisfy contingent and specific needs. For example, the technical characteristics described in relation to an embodiment of the invention can be extrapolated therefrom and applied to other embodiments of the invention. Such modifications and variations are moreover contained within the scope of the invention as defined by the following claims.

## Claims

1. A system (1) for monitoring exposure to radiation in a hospital environment, comprising:
- a dosimeter (2) wearable by a health care worker (O), configured to measure the ionizing radiation (MR) to which the worker (O) is exposed and comprising an identification number (ID)
- a radiation source identification means (4) for identifying a source of radiation emitted by an X-ray apparatus (3), comprising radiation source identification data (DCSR), the medical procedure (PRC) wherein the X-ray apparatus (3) is used and the position thereof within an area (A) of the hospital environment;
- a position acquisition means (5) for acquiring the position of the dosimeter (2), configured to communicate with said dosimeter (2) and to acquire the position in which it is located (POS);
- a radiation exposure monitoring unit (19) comprising:
• a first memory unit (26) comprising characteristic data of the health care worker (DCO) associated with said identification number (ID) of the dosimeter (2);
• a processing unit (20) configured to process data relating to the safety of each health care worker (O) comprising:
∘ an input module (21) configured to receive:
▪ from said dosimeter (2), the identification number (ID) and the measurement of ionizing radiation (MR) to which he is exposed;
▪ from said radiation source identification means (4), said characteristic data of the source of radiation emitted (DCSR) by the X-ray apparatus (3), the medical procedure (PRC) and the position (PA);
▪ from said position acquisition means (5), the position in which the dosimeter (2) is located (POS);
∘ a comparison module (23) configured to compare the data received by the input module (21) with predefined values;
∘ a conformity module (24) configured to generate a conformity signal as a function of a correspondence (OK) that has occurred in the comparison performed by said comparison module (23)
- a detection module (28) for detecting the personal protective equipment worn by the worker (O), configured to detect whether the worker (O) is wearing one or more items of personal protective equipment.
wherein the monitoring system (1) comprises a means for transmitting information to a remote server (30).

2. The system (1) according to claim 1, comprising a display module (25) configured to generate a map of the radiation doses absorbed by the worker (O) in the various positions within the area (A), and for each medical procedure (PRC).

3. The system (1) according to claim 1 or 2, wherein the processing unit (20) comprises a calculation module (22) configured to calculate a time of exposure (TES) of the worker (O) to the radiation and/or the relative distance (REL) between the position (POS) of the dosimeter (2) and the X-ray apparatus (3) and/or the measurement of the radiation received (MR).

4. The system (1) according to claim 3, wherein said comparison module (23) is configured to compare said time of exposure (TES) and/or said relative distance (REL) with predefined values.

5. The system according to one or more of the preceding claims, wherein the dosimeter (2) wearable by the worker (O) is associated with a detection device (6) for detecting biometric data (DBI).

6. The system according to claim 5, wherein said processing unit (20) is configured to perform a comparison between at least one item of biometric data (DBI) of the health care worker and the characteristic data of the worker (DCO) at the moment when the dosimeter (2) is donned.

7. The system according to one or more of the preceding claims, wherein the position acquisition means (5) for acquiring the position of the dosimeter (2) comprises one or more between, at least:
∘ time difference of arrival;
∘ two way ranging.

8. The system according to one or more of the preceding claims, wherein the position of the worker (O) is given by a location tag positioned on the dosimeter (2) or on the personal identification badge of the worker (O).

9. The system according to one or more of the preceding claims, wherein said characteristic data of the worker (DCO) comprise one or more among, at least:
∘ personal data;
∘ identification data;
∘ biometric data;
∘ health data.

10. The system according to one or more of the preceding claims, wherein said characteristic data of the source of radiation emitted (DCSR) comprise one or more among, at least:
∘ identification data;
∘ data on emissions of ionizing radiation.

11. The system according to one or more of the preceding claims, wherein placed in proximity to the zone of access to the area (A) there is an identification device (28) configured to identify the dosimeter (2) or the worker (O) entering or exiting the hospital area (A) or the personal identification badge.

12. The system according to one or more of the preceding claims, wherein said radiation source identification means (4) consists of an RFID radio-frequency tag and/or an NFC tag containing said characteristic data of the source of radiation emitted (DCSR).

13. A method for monitoring exposure to radiation in a hospital environment, comprising the steps of:
- assigning a wearable dosimeter (2) to a health care worker (O), the dosimeter being configured to measure the radiation (MR) to which the worker (O) is exposed and associable with the characteristic data of the health care worker (DCO) by means of a unique identification number (ID);
- preparing, on an X-ray apparatus (3) a radiation source identification means (4) for identifying a source of radiation emitted by the apparatus (3) comprising data identifying the source of radiation emitted (DCSR) and the position of the apparatus (3) within an area (A) of the hospital environment, as well as the medical procedures in which the X-ray apparatus (3)is used (PRC);
- preparing position acquisition means (5) for acquiring the position of the dosimeter (2), configured to communicate with said dosimeter (2) and to acquire the position wherein it is located (POS);
• receive said characteristic data of the health care worker (DCO) and the measurement by said dosimeter (2) of the ionizing radiation (MR) to which he is exposed;
• receive said characteristic data of the source of radiation emitted (DCSR) by the X-ray apparatus (3) from said means for identifying a source of radiation (4);
• receive the position in which (POS) the dosimeter (2) is located from said position acquisition means (5);
• compare the data received by the input module (21) with predefined values;
• generate a conformity signal as a function of a correspondence (OK) that has occurred in the comparison performed;
• transmit the information to a remote server (30) via an information transmission means;
- providing a detection module (28) for detecting the personal protective equipment worn by the worker (O), configured to detect whether the worker (O) is wearing one or more items of personal protective equipment.

## Patentansprüche

1. System (1) zur Überwachung der Strahlenbelastung in einer Krankenhausumgebung, umfassend:
- ein von einem Pflegemitarbeiter (O) tragbares Dosimeter (2), das zur Messung der ionisierenden Strahlung (MR), mit der der Pflegemitarbeiter (O) belastet wird, ausgelegt ist und eine Identifikationsnummer (ID) umfasst;
- Strahlungsquellenidentifikationsmittel (4) zum Identifizieren einer Strahlungsquelle, die von einem Röntgengerät (3) emittiert wird, umfassend Strahlungsquellenidentifikationsdaten (DCSR), das medizinische Verfahren (PRC), bei dem das Röntgengerät (3) verwendet wird, und dessen Position innerhalb des Bereichs (A) der Krankenhausumgebung;
- Positionserfassungsmittel (5) zum Erfassen der Position des Dosimeters (2), die ausgelegt sind, um mit dem Dosimeter (2) zu kommunizieren und die Position zu erfassen, in der es sich befindet (POS);
- eine Strahlungsbelastungsüberwachungseinheit (19), umfassend:
• eine erste Speichereinheit (26), die charakteristische Daten des Pflegemitarbeiters (DCO) umfasst, die mit der Identifikationsnummer (ID) des Dosimeters (2) assoziiert sind;
• eine Verarbeitungseinheit (20), die ausgelegt ist, um Daten in Bezug auf die Sicherheit eines jeden Pflegemitarbeiters (O) zu verarbeiten, umfassend:
∘ ein Eingabemodul (21), das ausgelegt ist, zu empfangen:
• aus dem Dosimeter (2) die Identifikationsnummer (ID) und die Messung der ionisierenden Strahlung (MR), mit der er belastet wird;
• aus den Strahlungsquellenidentifikationsmitteln (4) die charakteristischen Daten der von dem Röntgengerät (3) emittierten Strahlungsquelle (DCSR), das medizinische Verfahren (PRC) und die Position (PA);
• aus den Positionserfassungsmitteln (5) die Position, in der sich das Dosimeter (2) befindet (POS);
∘ ein Vergleichsmodul (23), das ausgelegt ist, um die von dem Eingabemodul (21) empfangenen Daten mit vordefinierten Werten zu vergleichen;
∘ ein Konformitätsmodul (24), das ausgelegt ist, um ein Konformitätssignal in Abhängigkeit von einer Übereinstimmung (OK) zu erzeugen, die bei dem von dem Vergleichsmodul (23) durchgeführten Vergleich aufgetreten ist;
- ein Detektionsmodul (28) zum Detektieren der von dem Mitarbeiter (O) getragenen persönlichen Schutzausrüstung, das ausgelegt ist, um zu detektieren, ob der Mitarbeiter (O) ein oder mehrere persönliche Schutzausrüstungsgegenstände trägt,
wobei das Überwachungssystem (1) Mittel zum Übertragen von Informationen an einen entfernten Server (30) umfasst.

2. System (1) nach Anspruch 1, umfassend ein Anzeigemodul (25), das ausgelegt ist, um eine Karte der Strahlungsdosierungen zu erzeugen, die von dem Mitarbeiter (O) in den verschiedenen Positionen innerhalb des Bereichs (A) und für ein jedes medizinische Verfahren (PRC) absorbiert werden.

3. System (1) nach Anspruch 1 oder 2, wobei die Verarbeitungseinheit (20) ein Berechnungsmodul (22) umfasst, das dazu ausgelegt ist, eine Belastungszeit (TES) des Mitarbeiters (O) gegenüber der Strahlung und/oder den relativen Abstand (REL) zwischen der Position (POS) des Dosimeters (2) und des Röntgengeräts (3) und/oder die Messung der empfangenen Strahlung (MR) zu berechnen.

4. System (1) nach Anspruch 3, wobei das Vergleichsmodul (23) ausgelegt ist, um die Belastungszeit (TES) und/oder den relativen Abstand (REL) mit vordefinierten Werten zu vergleichen.

5. System nach einem oder mehreren der vorhergehenden Ansprüche, wobei mit dem von dem Mitarbeiter (O) tragbaren Dosimeter (2) eine Detektionsvorrichtung (6) zum Detektieren biometrischer Daten (DBI) assoziiert ist.

6. System nach Anspruch 5, wobei die Verarbeitungseinheit (20) dazu ausgelegt ist, einen Vergleich zwischen mindestens einem biometrischen Datenelement (DBI) des Pflegemitarbeiters und den charakteristischen Daten des Mitarbeiters (DCO) in dem Moment durchzuführen, in dem das Dosimeter (2) aufgesetzt wird.

7. System nach einem oder mehreren der vorhergehenden Ansprüche, wobei die Positionserfassungsmittel (5) zum Erfassen der Position des Dosimeters (2) eine oder mehrere umfasst zwischen mindestens:
∘ Zeitdifferenz der Ankunft;
∘ Zwei-Wege-Entfernung.

8. System nach einem oder mehreren der vorhergehenden Ansprüche, wobei die Position des Mitarbeiters (O) durch ein auf dem Dosimeter (2) oder auf dem Personalausweis-Badge des Mitarbeiters (O) positioniertes Standort-Tag gegeben ist.

9. System nach einem oder mehreren der vorhergehenden Ansprüche, wobei die charakteristischen Daten des Mitarbeiters (DCO) einen oder mehrere davon mindestens umfassen:
∘ personenbezogene Daten;
∘ Identifikationsdaten;
∘ biometrische Daten;
∘ Gesundheitsdaten.

10. System nach einem oder mehreren der vorhergehenden Ansprüche, wobei die charakteristischen Daten der emittierten Strahlungsquelle (DCSR) eine oder mehrere davon mindestens umfassen:
∘ Identifikationsdaten;
∘ Daten über Emissionen ionisierender Strahlung.

11. System nach einem oder mehreren der vorhergehenden Ansprüche, wobei in der Nähe der Zone des Zugangs zu dem Bereich (A) eine Identifikationsvorrichtung (28) vorhanden ist, die ausgelegt ist, um das Dosimeter (2) oder den Mitarbeiter (O), der den Krankenhausbereich (A) betritt oder verlässt, oder das Personalausweis-Badge zu identifizieren.

12. System nach einem oder mehreren der vorhergehenden Ansprüche, wobei die Strahlungsquellenidentifikationsmittel (4) aus einem RFID-Hochfrequenz-Tag und/oder einem NFC-Tag bestehen, das/die die charakteristischen Daten der emittierten Strahlungsquelle (DCSR) enthält/enthalten.

13. Verfahren zur Überwachung der Strahlenbelastung in einer Krankenhausumgebung, umfassend die folgenden Schritte:
- Zuweisen eines tragbaren Dosimeters (2) an einen Pflegemitarbeiter (O), wobei das Dosimeter ausgelegt ist, um die Strahlung (MR) zu messen, mit der der Pflegemitarbeiter (O) belastet wird und die mit den charakteristischen Daten des Pflegemitarbeiters (DCO) mittels einer eindeutigen Identifikationsnummer (ID) assoziierbar ist;
- Vorbereiten von Strahlungsquellenidentifikationsmitteln (4) an einem Röntgengerät (3) zum Identifizieren einer Strahlungsquelle, die von dem Gerät (3) emittiert wird, umfassend Daten, die die emittierte Strahlungsquelle (DCSR) und die Position des Geräts (3) innerhalb eines Bereichs (A) der Krankenhausumgebung sowie die medizinischen Verfahren, in denen das Röntgengerät (3) verwendet wird (PRC), identifizieren;
- Vorbereiten von Positionserfassungsmitteln (5) zum Erfassen der Position des Dosimeters (2), die ausgelegt sind, um mit dem Dosimeter (2) zu kommunizieren und die Position zu erfassen, in der es sich befindet (POS);
• Empfangen der charakteristischen Daten des Pflegemitarbeiters (DCO) und der Messung der ionisierenden Strahlung (MR), mit der er belastet wird, durch das Dosimeter (2);
• Empfangen der charakteristischen Daten der von dem Röntgengerät (3) emittierten Strahlungsquelle (DCSR) von den Mitteln zum Identifizieren einer Strahlungsquelle (4);
• Empfangen der Position, in der sich (POS) das Dosimeter (2) befindet, von den Positionserfassungsmitteln (5);
• Vergleichen der vom Eingabemodul (21) empfangenen Daten mit vordefinierten Werten;
• Erzeugen eines Konformitätssignals in Abhängigkeit von einer Übereinstimmung (OK), die bei dem durchgeführten Vergleich aufgetreten ist;
• Übertragen der Informationen an einen entfernten Server (30) über Informationsübertragungsmittel;
- Bereitstellen eines Detektionsmoduls (28) zum Detektieren der von dem Mitarbeiter (O) getragenen persönlichen Schutzausrüstung, das ausgelegt ist, um zu detektieren, ob der Mitarbeiter (O) ein oder mehrere persönliche Schutzausrüstungsgegenstände trägt.

## Revendications

1. Système (1) de surveillance de l'exposition au rayonnement dans un environnement hospitalier, comprenant :
- un dosimètre (2) pouvant être porté par un professionnel de santé (O), configuré pour mesurer le rayonnement ionisant (MR) auquel le professionnel (O) est exposé et comprenant un numéro d'identification (ID) ;
- des moyens d'identification (4) de source de rayonnement servant à identifier une source de rayonnement émise par un appareil à rayons X (3), comprenant des données d'identification de source de rayonnement (DCSR), la procédure médicale (PRC) dans laquelle l'appareil à rayons X (3) est utilisé et la position de celui-ci au sein d'une zone (A) de l'environnement hospitalier ;
- des moyens d'acquisition de position (5) servant à acquérir la position du dosimètre (2), configurés pour communiquer avec ledit dosimètre (2) et pour acquérir la position dans laquelle il se trouve (POS) ;
- une unité de surveillance (19) de l'exposition au rayonnement comprenant :
• une première unité de mémoire (26) comprenant des données spécifiques du professionnel de santé (DCO) associées audit numéro d'identification (ID) du dosimètre (2) ;
• une unité de traitement (20) configurée pour traiter les données relatives à la sécurité de chaque professionnel de santé (O) comprenant :
∘ un module d'entrée (21) configuré pour recevoir :
• à partir dudit dosimètre (2), le numéro d'identification (ID) et la mesure du rayonnement ionisant (MR) auquel il est exposé ;
• à partir desdits moyens d'identification (4) de la source de rayonnement, lesdites données spécifiques de la source de rayonnement émise (DCSR) par l'appareil à rayons X (3), la procédure médicale (PRC) et la position (PA) ;
• à partir desdits moyens d'acquisition de position (5), la position dans laquelle le dosimètre (2) se trouve (POS) ;
∘ un module de comparaison (23) configuré pour comparer les données reçues par le module d'entrée (21) avec des valeurs prédéfinies ;
∘ un module de conformité (24) configuré pour générer un signal de conformité en fonction d'une correspondance (OK) qui s'est produite lors de la comparaison effectuée par ledit module de comparaison (23) ;
- un module de détection (28) servant à détecter l'équipement de protection individuelle porté par le professionnel (O), configuré pour détecter si le professionnel (O) porte un ou plusieurs articles d'équipement de protection individuelle,
dans lequel le système de surveillance (1) comprend des moyens servant à transmettre des informations à un serveur distant (30).

2. Système (1) selon la revendication 1, comprenant un module d'affichage (25) configuré pour générer une carte des doses de rayonnement absorbées par le professionnel (O) dans les différentes positions à l'intérieur de la zone (A), et pour chaque procédure médicale (PRC).

3. Système (1) selon la revendication 1 ou 2, dans lequel l'unité de traitement (20) comprend un module de calcul (22) configuré pour calculer une durée d'exposition (TES) du professionnel (O) au rayonnement et/ou la distance relative (REL) entre la position (POS) du dosimètre (2) et l'appareil à rayons X (3) et/ou la mesure du rayonnement reçu (MR).

4. Système (1) selon la revendication 3, dans lequel ledit module de comparaison (23) est configuré pour comparer ladite durée d'exposition (TES) et/ou ladite distance relative (REL) avec des valeurs prédéfinies.

5. Système selon l'une ou plusieurs des revendications précédentes, dans lequel le dosimètre (2) pouvant être porté par le professionnel (O) est associé à un dispositif de détection (6) servant à détecter des données biométriques (DBI).

6. Système selon la revendication 5, dans lequel ladite unité de traitement (20) est configurée pour effectuer une comparaison entre au moins un élément de données biométriques (DBI) du professionnel de santé et les données spécifiques du professionnel (DCO) au moment où le dosimètre (2) est porté.

7. Système selon l'une ou plusieurs des revendications précédentes, dans lequel les moyens d'acquisition de position (5) servant à acquérir la position du dosimètre (2) comprend une ou plusieurs caractéristiques parmi, au moins :
∘ la différence de temps d'arrivée ;
∘ la télémétrie bidirectionnelle.

8. Système selon l'une ou plusieurs des revendications précédentes, dans lequel la position du professionnel (O) est donnée par une balise de localisation positionnée sur le dosimètre (2) ou sur le badge d'identification personnel du professionnel (O).

9. Système selon l'une ou plusieurs des revendications précédentes, dans lequel lesdites données spécifiques du professionnel (DCO) comprennent au moins une ou plusieurs des données parmi :
∘ les données à caractère personnel ;
∘ les données d'identification ;
∘ les données biométriques ;
∘ les données de santé.

10. Système selon l'une ou plusieurs des revendications précédentes, dans lequel lesdites données caractéristiques de la source de rayonnement émis (DCSR) comprennent au moins une ou plusieurs des données parmi :
∘ les données d'identification ;
∘ les données sur les émissions du rayonnement ionisant.

11. Système selon l'une ou plusieurs des revendications précédentes, dans lequel, placé à proximité de la zone d'accès à la zone (A), se trouve un dispositif d'identification (28) configuré pour identifier le dosimètre (2) ou le professionnel (O) entrant ou sortant de la zone hospitalière (A) ou le badge d'identification personnel.

12. Système selon l'une ou plusieurs des revendications précédentes, dans lequel lesdits moyens d'identification (4) de source de rayonnement consiste en une étiquette radiofréquence RFID et/ou une étiquette utilisant la communication en champ proche (NFC) contenant lesdites données caractéristiques de la source de rayonnement émise (DCSR).

13. Procédé de surveillance de l'exposition au rayonnement dans un environnement hospitalier, comprenant les étapes suivantes :
- attribuer un dosimètre portable (2) à un professionnel de santé (O), le dosimètre étant configuré pour mesurer le rayonnement (MR) auquel le professionnel (O) est exposé et pouvant être associé aux données spécifique du professionnel de santé (DCO) au moyen d'un numéro d'identification unique (ID) ;
- préparer, sur un appareil à rayons X (3), des moyens d'identification (4) de source de rayonnement pour identifier une source de rayonnement émise par l'appareil (3), comprenant des données identifiant la source de rayonnement émise (DCSR) et la position de l'appareil (3) à l'intérieur d'une zone (A) de l'environnement hospitalier, ainsi que les procédures médicales dans lesquelles l'appareil à rayons X (3) est utilisé (PRC) ;
- préparer des moyens d'acquisition de position (5) servant à acquérir la position du dosimètre (2), configurés pour communiquer avec ledit dosimètre (2) et pour acquérir la position dans laquelle il se trouve (POS) ;
• recevoir lesdites données spécifiques du professionnel de santé (DCO) et la mesure par ledit dosimètre (2) du rayonnement ionisant (MR) auquel il est exposé ;
• recevoir lesdites données caractéristiques de la source de rayonnement émise (DCSR) par l'appareil à rayons X (3) à partir desdits moyens d'identification (4) d'une source de rayonnement ;
• recevoir la position dans laquelle (POS) se trouve le dosimètre (2) à partir desdits moyens d'acquisition de position (5) ;
• comparer les données reçues par le module d'entrée (21) à des valeurs prédéfinies ;
• générer un signal de conformité en fonction d'une correspondance (OK) qui s'est produite lors de la comparaison effectuée ;
• transmettre les informations à un serveur distant (30) via des moyens de transmission d'informations ;
- fournir un module de détection (28) servant à détecter l'équipement de protection individuelle porté par le professionnel (O), configuré pour détecter si le professionnel (O) porte un ou plusieurs articles d'équipement de protection individuelle.
